# EUROPEAN PATENT APPLICATION

(11) **EP 4 316 575 A1**
(43) Date of publication of application: **07.02.2024**
(21) Application number: 22780547.0
(22) Date of filing: 25.03.2022
(51) Int. Cl.: A61N 2/08

(54) **STELLATE GANGLION MAGNETIC STIMULATION DEVICE, AND METHODS FOR WEARING AND USING SAME**

(30) Priority: 30.03.2021 JP 2021057239
(71) Applicant: OSAKA UNIVERSITY, Suita-shi Osaka 565-0871 (JP)
(72) Inventor: YAGI Masakazu, Suita-shi, Osaka 565-0871 (JP); SAWA Yoshiki, Suita-shi, Osaka 565-0871 (JP); MASUDA Hirotada, Suita-shi, Osaka 565-0871 (JP); YOSHIDA Kotaro, Suita-shi, Osaka 565-0871 (JP); MINAKAMI Fumito, Suita-shi, Osaka 565-0871 (JP); KANEDA Eri, Suita-shi, Osaka 565-0871 (JP)
(74) Representative: Mewburn Ellis LLP
(86) International application number: PCT/JP2022/014295
(87) International publication number: WO 2022/210333

(57) **Abstract**

In order to provide a technical means that offers excellent convenience, usability, and safety for stellate ganglion block that can effectively suppress sympathetic overexcitation, this stellate ganglion magnetic stimulation device comprises: a head unit having a magnet for generating magnetism; and a neck unit that is removably worn around a wearer's neck and that presses the head unit against epidermis corresponding to the stellate ganglion so that stimulation by the magnetism acts on the stellate ganglion.

## Description

### TECHNICAL FIELD

The present invention relates to a stellate ganglion magnetic stimulation device for magnetically stimulating the stellate ganglion, and a method of wearing and using the same.

### BACKGRAOUND ART

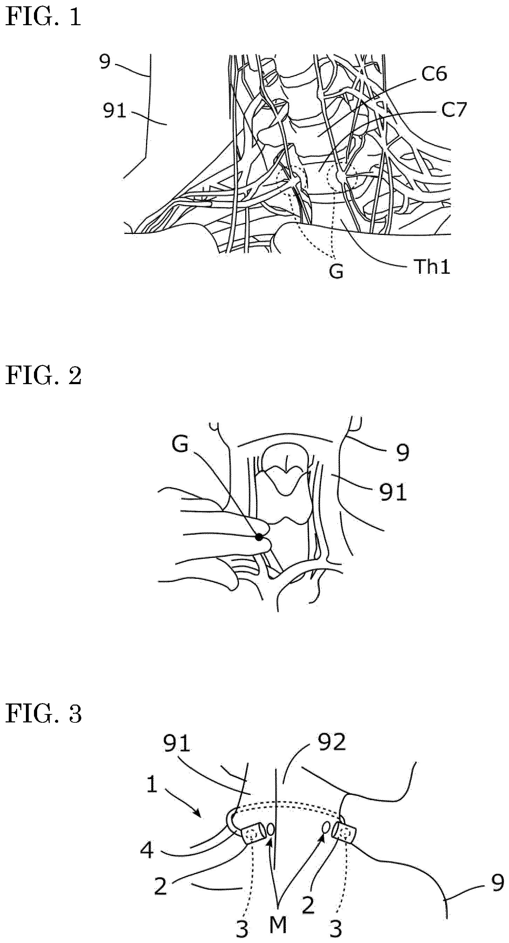

A ganglion is a mass of nerve cell bodies in the peripheral nervous system, and sympathetic nerves which control blood circulation in the upper body are concentrated in the stellate ganglion (see FIG. 1). Sympathetic overexcitation is known to cause various symptoms such as hot flash and chronic pain.

Various methods have been attempted to suppress the sympathetic nerves in order to reduce or eliminate the specific symptoms associated with sympathetic overexcitation. For example, Patent Document 1 describes that stellate ganglion block injection relieves chronic pain and the like.

For example, Non Patent Document 1 proposes a technique for reducing the hot flash by wearing a headgear-type device including an anode and a cathode on the head and applying electrical stimulation to transcranial.

### PRIOR ART DOCUMENT

### PATENT DOCUMENT

Patent Document 1: Utility Model No. 3101426

### NON-PATENT DOCUMENT

Non-Patent Document 1: Transcranial direct current stimulation effects on menopausal vasomotor symptoms, Menopause: The Journal of The North American Menopause Society, Vol. 24, No. 10

### SUMMARY OF THE INVENTION

### PROBLEM TO BE SOLVED BY THE INVENTION

However, the stellate ganglion block injection proposed in Patent Document 1 cannot be readily used outside of medical institutions, and there is a risk of side effects and infection due to direct insertion of the needle into the body. Further, in the technique proposed in Non-Patent Document 1, since the anode and the cathode must be placed in the motor cortex region and the supraorbital region of the cerebral hemisphere, respectively, the feeling of use is poor, and it is said that the effect is not necessarily sufficient.

Accordingly, an object of the present invention is to provide a technical means that offers excellent convenience, usability, and safety for stellate ganglion block that can effectively suppress sympathetic overexcitation.

### MEANS TO SOLVE THE PROBLEMS

In order to solve the above-mentioned problems, the present invention provides a stellate ganglion magnetic stimulation device, which includes:
a head unit having a magnet for generating magnetism; and
a neck unit that is detachably worn around a neck portion, ear portion or head portion of a wearer and that presses the head unit against epidermis corresponding to the stellate ganglion so that stimulation by the magnetism acts on the stellate ganglion.

In the stellate ganglion magnetic stimulation device of the present invention, it is preferable that the magnet includes at least two magnet pieces, and
the at least two magnet pieces are so arranged as to satisfy the equation of 30°≤α≤180° where a is the angle between their axes.

In the stellate ganglion magnetic stimulation device of the present invention, it is preferable that the magnet includes at least three magnet pieces, and
the at least three magnet pieces are so arranged as to satisfy the equations of 30°≤β≤180° and 30°≤γ≤180° where 6 and y are the angles between the axes of adjacent magnet pieces.

In the stellate ganglion magnetic stimulation device of the present invention, it is preferable that the magnet generates a magnetic field such that a magnetic flux density of 20 mT or more reaches the corresponding stellate ganglion.

Further, in the stellate ganglion magnetic stimulation device of the present invention, it is preferable that the neck unit has an expansion and contraction mechanism for expanding and contracting the neck unit along the circumference of the neck portion.

Further, the present invention also provides a method of wearing or a method of using the stellate ganglion magnetic stimulation device of the present invention, which includes the steps of
determining the position of the stellate ganglion based on the position of at least one of the seventh cervical vertebra and the first thoracic vertebra of the wearer, and
marking the epidermis corresponding to the determined position of the stellate ganglion,
wearing the neck unit around the neck portion, and
arranging the head unit according to the mark.

### EFFECT OF THE INVENTION

According to the present invention, it is possible to provide a technical means that offers excellent convenience, usability, and safety for stellate ganglion block that can effectively suppress sympathetic overexcitation.

### BRIEF DESCRIPTION OF THE DRAWINGS

FIG. 1 is an explanatory diagram of the stellate ganglion G.
FIG. 2 is a schematic diagram showing the position of the stellate ganglion G.
FIG. 3 is a schematic diagram of the stellate ganglion magnetic stimulation device 1.
FIG. 4 is a diagram showing a specific configuration example of the magnets 3.
FIG. 5 is a diagram showing a specific configuration example of the head unit 2.
FIG. 6 is a diagram showing other specific configuration example of the magnets 3.
FIG. 7 is a diagram showing a specific configuration example of the neck unit 4.
FIG. 8 is a measurement result (graph) showing the activity of the sympathetic nerve and the parasympathetic nerve when the magnet is worn to the left neck portion and when the magnet is not worn.
FIG. 9 is a diagram explaining the order arrangement and the reverse arrangement of the magnet pieces 31 and 32.
FIG. 10 is a diagram explaining the positional relationship between the magnets M1 and M2 in the animal experiment 2.

### MODE FOR CARRYING OUT THE INVENTION

In the following, by referring the drawings, embodiments according to the stellate ganglion magnetic stimulation device of the present invention, and the wearing method and the using method thereof explained, but the present invention is not limited thereto. In addition, since these drawings are presented to explain the concept of the present invention, the sizes, ratios or numbers may be exaggerated or simplified for easy understanding.

There are individual differences in the origin and terminal of the three cervical ganglia, including the stellate ganglion. The stellate ganglion G, as shown in FIG. 1, is located approximately at the level of the seventh cervical vertebrae (C7) to the second thoracic vertebrae (Th2) and is thinly attached to the bone. For example, the approximate position of the stellate ganglion G can be determined as follows (see FIG. 2).

That is, the patient (he/she), who is the wearer of the stellate ganglion magnetic stimulation device according to the present invention, looks down and checks the seventh cervical vertebra (C7), which protrudes behind the neck. And then, the patient touches the sternocleidomastoid muscle 92 (the muscle that originates at the sternum and clavicle and terminates at the mastoid process of the temporal bone and the occipital bone). The stellate ganglion G is located generally anteriorly or posteriorly to the sternocleidomastoid muscle 92 at the intersection (closest) between the sternocleidomastoid muscle 92 and the seventh cervical vertebra (C7). However, this is an empirical method of stellate ganglion identification and does not imply anatomical accuracy.

In order to efficiently deliver a magnetic force to such a stellate ganglion G and perform magnetic stimulation, a stellate ganglion magnetic stimulation device 1 (hereinafter referred to as device 1) according to the present embodiment includes the head unit 2 and the neck unit 4. Each component will be described in detail below.

The head unit 2 has a magnet 3 that generates magnetism. The magnet 3 may be a permanent magnet that forms a static magnetic field or an electromagnet that forms a dynamic magnetic field, but the case of the permanent magnet will be explained here. Further, at least one head unit 2 is sufficient, but in the present embodiment, two head units 2 are provided corresponding to two stellate ganglia G.

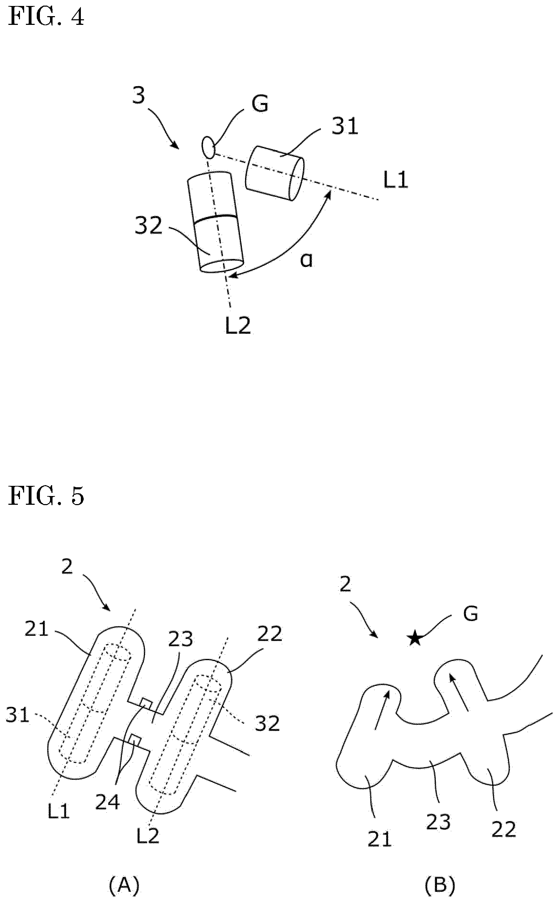

From the viewpoint of appropriately stimulating the stellate ganglion G, each head unit 2 preferably includes at least two magnet pieces 31 and 32 as the magnet 3 (see FIG. 4). The magnet pieces 31 and 32 are so arranged that, when the angle formed by their axes (that is, the lines indicating the direction in which the magnetic poles of the magnet pieces 31 and 32 are arranged) L1 and L2 is assumed to be a, the angle preferably satisfies the equation of 30°≤α≤180°, and more preferably equation of 60°≤α≤180°. Further, the upper limit of a may be 120°.

The magnet pieces 31 and 32 may have a columnar shape, or may have a cylindrical shape from the viewpoint of weight reduction. Further, the magnet pieces 31 and 32 may have substantially the same surface magnetic flux density, or one magnet segment (for example, the magnet segment 31) may have a higher surface magnetic flux density than the other magnet segment (for example, the magnet segment 32). In order to make the surface magnetic flux density higher, the number of magnet pieces may be increased, or strong magnet pieces may be used. For example, when increasing the number of magnet pieces, by arranging columnar or cylindrical magnets in series (or along the alignment direction of the magnetic poles) (see, for example, the magnet pieces 32 in FIG. 4), it is possible to give stimulation from all directions.

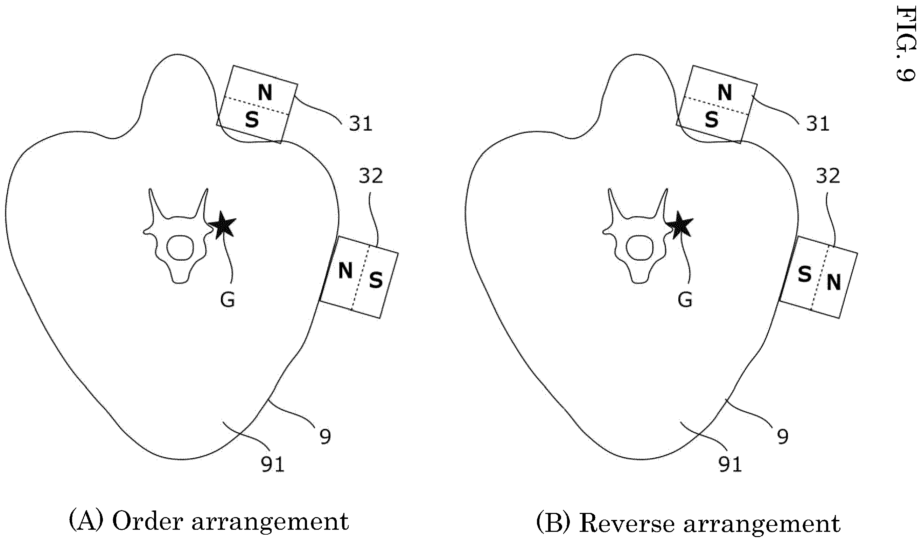

The magnetic pole arrangement of the magnet pieces 31 and 32 may be order arrangement or may be reverse arrangement. In this specification, the order arrangement means that the north pole of the magnet piece 31 and the south pole of the magnet piece 32 face the target (that is, the stellate ganglion G of the wearer 9) side, and the south pole of the magnet piece 31 and the north pole of the magnet piece 32 face the target side is called forward arrangement (see FIG. 9(A)). Further, the reverse arrangement means that the north poles of the magnet pieces 31 and 32 face the target side, and the south poles of the magnet pieces 31 and 32 face the target side (see FIG. 9(B)). By adopting the order arrangement, it is expected that a larger magnetic gradient can be obtained.

As shown in FIG. 5(A), the magnet pieces 31 and 32 are accommodated in the storage portions 21 and 22 of the head unit 2, respectively. By fixing the magnet pieces 31 and 32, the storage portions 21 and 22 also serve to prevent accidents caused by strongly attracting the magnet pieces each other.

The storage portions 21 and 22 are connected to each other via the connecting portion 23. The connecting portion 23 is made of, for example, a flexible resin material, and can change the irradiation direction of the magnetic force by the magnet pieces 31 and 32 (that is, the directions of the axes L1 and L2) by deforming (see FIG. 5(B)). Therefore, it is possible to efficiently irradiate the magnetic force of the magnet pieces 31 and 32 toward the stellate ganglion G as a target.

For example, when the storage portions 21 and 22 of the head unit 2 are composed of a storage space (not shown) for the magnet pieces 31 and 32 and a lid (not shown) for closing the storage space, the head unit 2 may have at least one pair of claws 24. The claws 24 are provided, for example, on the opposite surfaces (for example, the upper surface and the lower surface) of the connecting portion 23, and by fitting the upper and lower claws 24, the lid is fixed, and the magnet pieces 31 and 32 can be securely stored into the storage portions 21 and 22.

In addition, not shown, a rail or slide mechanism may be provided on the connecting portion 23 or the like so that the storage portions 21 and 22 can move relative to each other.

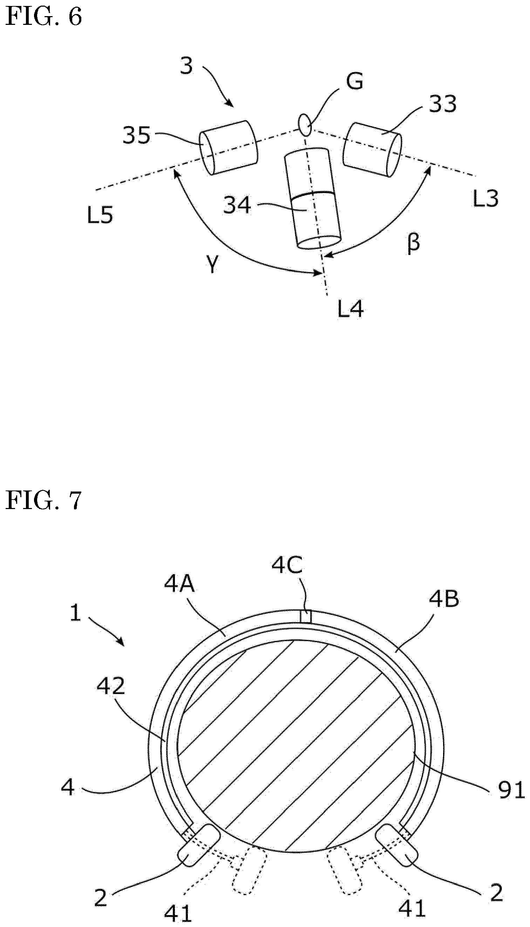

Alternatively, in order to stimulate the stellate ganglion G more strongly or more efficiently, each head unit 2 may include at least three magnet pieces 33 to 35 as the magnet 3 (see FIG. 6). The magnet pieces 33 to 35 are preferably arranged so as to satisfy the equations of 30°≤β≤180° and 30°≤γ≤180°, where 6 and y are the angles formed by the adjacent axes L3 to L5, and above all, it is preferable that they are arranged so as to satisfy the equations of 60°≤β≤120° and 60°≤γ≤120°. The upper limits of 6 and y may each be 120°. In this case, the number of storage portions included in the head unit 2 is at least three (not shown).

Alternatively, in order to avoid excessive stimulation of the parasympathetic nerves, each head unit 2 may include a single magnet piece as the magnet 3 (see FIG. 3).

A switch mechanism (not shown) for controlling the magnetic force of the magnet 3 may be provided. When the magnet 3 is an electromagnet, the switch mechanism is a switch or circuit which controls the current through the coil. Further, when the magnet 3 is a permanent magnet, the magnetic force of the magnet 3 is controlled by inserting, for example, a sheet for controlling the magnetic field into a device which holds the magnet 3 or the storage portions 21 and 22.

It is desirable that the magnets 3 are so appropriately adjusted that a predetermined magnetic flux density reaches the target stellate ganglion G within the range in which the effects of the present invention can be obtained. The lower limit of the magnetic flux density to be reached may be 20 mT, preferably 40 mT. Further, the upper limit of the magnetic flux density to be reached may be 150 mT, preferably 60 mT. As the magnet 3, for example, a neodymium magnet, a samarium-cobalt magnet, or a ferrite magnet can be preferably used. In addition, a soft magnetic material such as permalloy may be used together with the magnet.

For example, as shown in FIG. 3, the neck unit 4 is a ring-shaped member detachably worn around the neck portion 91 of the wearer 9 to hold the device 1 in a desired position on the neck portion 91. In order to act the magnetic field generated by the magnet 3 on the stellate ganglion G, the neck unit 4 presses the head unit 2 against the epidermis corresponding to the stellate ganglion G. In the present embodiment, the head units 2 are arranged at both ends of the neck unit 4. An additional head unit 2 may be provided at other portion of the neck unit 4. The neck unit 4 may be worn around an ear portion or a head portion of the wearer 9.

As shown in FIG. 7, it is preferable that the neck unit 4 has an expansion and contraction mechanism 41 that allows the neck unit 4 to expand and contract around the neck portion 91. Thereby, regardless of the size of the neck portion 91 of the wearer 9, it becomes easy to appropriately arrange the head unit 2 on the stellate ganglion G, and the stellate ganglion G can be sufficiently stimulated.

As the expansion and contraction mechanism 41, for example, a rod-shaped or plate-shaped member that is slidably accommodated in the neck unit 4 or pulled out from the neck unit 4 can be suitably used, but the expansion and contraction mechanism 41 is not limited thereto.

The neck unit 4 may have a cushion 42 on the surface facing the neck portion 91 of the wearer 9. Due to the damping action of the cushion, the burden on the neck portion 91 of the wearer 9 can be reduced.

Further, the neck unit 4 may be configured, for example, by connecting a plurality of rod-like or arm-like members 4A, 4B, and in that case, for example, a hinge 4C may connect in the foldable manner.

Next, the procedures for wearing or using the device 1 will be described.

First, the position of the stellate ganglion G is determined (or guessed) as accurately as possible. For example, the position of the stellate ganglion G can be determined from the position information of the 6th and 7th cervical vertebrae (C6, C7) obtained by neck echo. Alternatively, by making the wearer 9 look down, the seventh cervical vertebra (C7) that protrudes behind the neck is checked. By touching the sternocleidomastoid muscle 92, it is possible to determine that the position of the stellate ganglion G is the anterior or posterior position of the sternocleidomastoid muscle 92 at the intersection between the sternocleidomastoid muscle 92 and the seventh cervical vertebra (C7).

As a method or procedure for evaluating the adequacy of the wearing position of the magnet 3, it is possible to use a physiological index reflecting the state of the sympathetic nerves, as typified by the heart rate variability analysis system described later. For example, it is possible to construct a system that senses the above physiological index in a relatively short period of time and displays the sensed physiological index. The system may automatically start the sensing of the above-described physiological index, and determine whether the wearing position of the magnet 3 is appropriate on the basis of the temporal change in the obtained physiological index.

A specific example is an earphone-type device that can measure peripheral blood flow. By linking with this device, it is possible to confirm the certainty of the wearing position of the magnet 3, and furthermore, it is possible to measure the effect of the device 1.

Alternatively, a blood flow meter can be used. By obtaining waveforms and sounds peculiar to arteries with the blood flow meter, it is possible to determine an appropriate wearing position of the magnet 3 from the size of the waveforms and sounds. Specifically, an ultrasonic blood flow meter (for example, HUNTLEIGH ultrasonic blood flow meter Dopplex DMX available from Muranaka Medical Instruments Co., Ltd.) can be preferably used.

Next, a mark M is made on the epidermis of the neck portion 91 corresponding to the estimated position of the stellate ganglion G. For example, the epidermis of the neck portion 91, which is the shortest distance from the estimated position of the stellate ganglion G, is marked M with a vegetable dye or the like. Two or more marks M may be used.

Then, the neck unit 4 of the device 1 is temporarily worn to the neck portion 91 of the wearer 9. As the wearing site, for example, a protruding bone behind the neck portion 91 of the wearer 9 is suitable.

The expansion and contraction mechanism of the neck unit 4 is adjusted to fit the neck unit 4 to the circumference of the neck portion 91. After that, the head part 2 is aligned with the mark M and arranged. At this time, the neck unit 4 preferably presses the head unit 2 against the neck portion 91 of the wearer 9. As a result, it is expected that the head part 2 will be fixed so as not to deviate from the stellate ganglion G, and that the magnetic field generated by the magnet 3 will sufficiently act on the stellate ganglion G.

Then, the magnetic stimulation from the neck unit 4 (magnet 3) acts on the stellate ganglion G, suppresses the overexcitation of the sympathetic nerves, and is expected to eliminate or reduce various symptoms. Presumed symptoms include symptoms of hot flashes and sweating (hot flashes) occurring during menopause.

Since the device 1 is compact, unobtrusive, and non-invasive, it is possible to be worn for extended periods of time, and to be worn when going out. Further, the device 1 is easy to use and safe. Furthermore, since the device 1 is easily portable, the device 1 can be started to use as soon as symptoms appear. Thus, the convenience of the device 1 is high.

### -Measurement example-

Here, the effectiveness of magnetic stimulation of the stellate ganglion G by the device 1, especially the permanent magnet, will be described.

How to act the autonomic nerves is evaluated by the heart rate variability analysis at the time when the magnet was applied to the left neck of a human being and at the time when it was not applied to the left neck. The heart rate variability analysis system used here is the analysis software called Reflex Meijin (trademark) of Crosswell Co., Ltd.

The permanent magnet used here is the following neodymium magnet (N40) available from Neomag Co., Ltd.
Shape: Cylindrical
Dimensions: 25 mm diameter x 15 mm height
Surface magnetic flux density: 476 mT
Magnetization direction: height direction

The permanent magnets used are two neodymium magnets arranged in series. The time for applying the permanent magnet is 20 minutes.

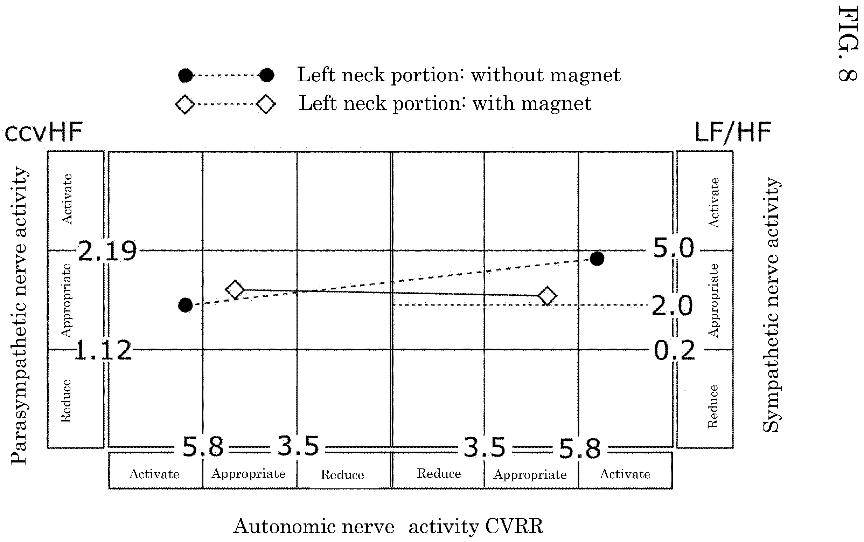

The analysis results are shown in FIG. 8. Here, HF is a high frequency component power of the measured waveform and LF is a low frequency component power of the waveform. CCV(HF) is a high frequency component of the fluctuation of the waveform and serves as a parasympathetic index. LF/HF is an index of balance between the sympathetic nerves and the parasympathetic nerves, and when the value is high, it is an index of sympathetic nerves.

From these analysis results, it was confirmed that, when the magnet was not worn to the left portion of the neck, the sympathetic nerve activity was slightly activated, but that, when the magnet was attached to the left portion of the neck, the sympathetic nerve activity was in an appropriate range.

### -Animal Experiments-

### (1) Wearing magnet to rat

Adrenergic agonist was intraperitoneally administered to a rat in an appropriate amount to raise blood pressure by 120 to 150% compared to normal state. At the time when the blood pressure of the rat reached 140%, a magnet was worn to the neck portion of the rat to observe the blood pressure of the rat. The wearing of the magnet was maintained for the next 45 minutes, and thereafter, the magnet was removed from the rat. After that, the blood pressure measurement of the rat was continued for 15 minutes, and the blood pressure was compared with that of a rat which was worn with a sham magnet (magnet giving physiologically meaningless stimulation) set as a control.

The permanent magnet used in this embodiment (hereinafter referred to as this magnet) is two neodymium magnets (N40) available from Neomag Co., Ltd. arranged in series. Each neodymium magnet is ring-shaped having an outer diameter of 43.9 mm, an inner diameter of 33.1 mm, and a height of 22 (mm).

The weight of the rat used is 220 g, and the distance from the surface of the neck portion to the stellate ganglion is estimated from "Atlas of Cross-Sectional Anatomy of Rat" published by Adthree Co., Ltd., and is 10 mm.

The magnetic field gradient of this magnet estimated from numerical analysis by the applicant is 7 mT/mm to 9 mT/mm.

Also, an aluminum bronze (C6191B) was used as the sham magnet. This metal is a copper alloy where iron, manganese, nickel, and the like are added to an alloy based on copper and aluminum. In this embodiment, two cylindrical copper alloys having a diameter of 40 mm and a length of 19 mm were arranged in series. The copper alloy is set approximately equal in weight to the neodymium magnet described above.

As a result of the experiment, a large drop in blood pressure was observed in the rats wearing this magnet compared to the control, and the blood pressure tended to drop even after this magnet was removed. That is, it was confirmed that the sympathetic nerve was suppressed by wearing this magnet.

### (2) Wearing magnets to pig

In the experiments using pigs, the invasive blood pressure measurement and peripheral skin blood flow amount measurement were set as observation items. The experiment was divided into four phases, i.e. (i) normal phase, (ii) at the administration of the adrenergic agonist (phenylephrine), (iii) 15 to 60 minutes after the wearing of the magnet, and (iv) at the removal of the magnet and cessation of administration of the adrenergic agonist, and the difference in effect between the Sham magnet and this magnet was investigated.

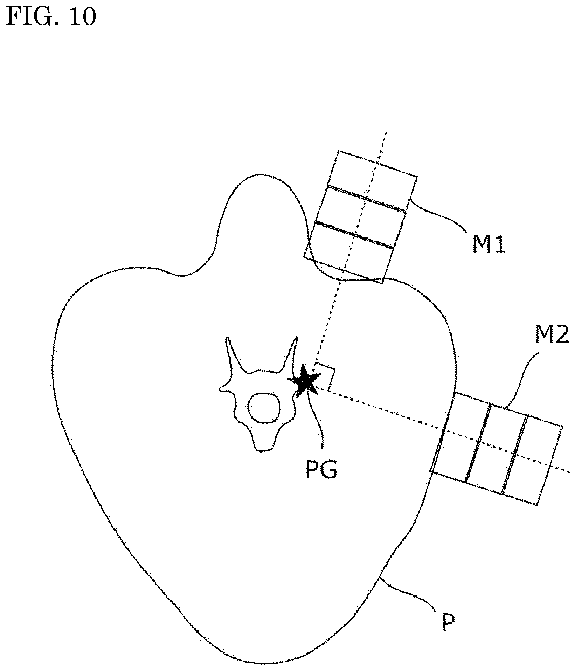

The permanent magnet used in this embodiment (hereinafter referred to as this magnet) is three neodymium magnets (N40) available from Neomag Co., Ltd. arranged in series. Each neodymium magnet is cylindrical, and has an outer diameter of 40 mm and a height of 20 mm. In this experiment, one set of this magnet M1, M2 was placed on the neck portion of the pig P so that their axes intersect at approximately 90 degrees (see FIG. 10).

The pig P used is a microminipig having a body weight of 10 to 14 kg, and the distance to the superficial stellate ganglion PG of the neck portion assumed from the dissection and CT images is 30 mm to 50 mm. Further, the magnetic field gradient of this magnet estimated from the numerical analysis by the applicant is 1.5 mT/mm to 2.5 mT/mm.

The results of the experiments are shown in the following Table 1.

**[Table 1]**

| Change rate (%) of average skin blood flow amount (z/W) of auricle *1 | Phase 1: | Phase 2: | Phase 3: | Phase 4: |
|---|---|---|---|---|
| | No admn. Pre | During admn. Pre | During admn. | No admn. |
| | | | 15-60 min. after wearing | No magnet wearing |
| Sham magnet | 100 | 96.2 | 86.0 | 85.6 |
| This magnet | 100 | 92.4 | 99.0 | 90.8 |

| Change rate (%) of systolic blood pressure (mmHg) of artery *2 | Phase 1: | Phase 2: | Phase 3: | Phase 4: |
|---|---|---|---|---|
| | No admn. Pre | During admn. Pre | During admn. | No admn. |
| | | | 15-60 min. after wearing | No magnet wearing |
| Sham magnet | 100 | 123.2 | 132.8 | 125.2 |
| This magnet | 100 | 136.9 | 142.7 | 142.7 |

| | | | | |
|---|---|---|---|---|
| *1: Usually reduce with drug administration. *2: Usually elevate with drug administration. | | | | |

As a result of the experiments, although the influence of wearing this magnet on the central blood pressure was small, the effect of the magnetic field on the stellate ganglion caused a change in the peripheral blood pressure, and it was considered that the effect resulted in a large recovery of the peripheral skin blood flow.

During the wearing of this magnet, the pulse-like changes in the amount of blood flow were observed several times, except for contamination of noise. It is considered that the reason is that, since healthy pigs were used in the experiments, at the time when the action of the vascular system and nervous system with normal functions for counteracting the changes which were caused by the administration of adrenergic agonists collapsed, such changes would be exhibited.

As for the short-term effect during 45 minutes of observation time, it was not effective enough to suppress the elevation of the central blood pressure, but it was observed that the amount of the blood flow reflecting the peripheral blood pressure was greatly influenced. That is, it was confirmed that the wearing of this magnet had an inhibitory effect on the sympathetic nerves that control the peripheral blood vessels. Long-term wearing for several days is expected to have the effect of suppressing elevation of central blood pressure.

In the experiment of wearing magnet to the rat, the generation of the magnetic field gradient at 7 mT/mm to 9 mT/mm in the stellate ganglion showed a clear tendency to lower the blood pressure. In the experiment of wearing magnet to the pig, the generation of the magnetic field gradient at 1.5 mT/mm to 2.5 mT/mm in the stellate ganglion showed a clear tendency to recover the blood flow.

It is known that in research on the effect of a static magnetic field on the brain, an effect of 1.5 mT/mm or more for 15 minutes or longer is the threshold for producing effects on the brain. According to the above experiments, it is suggested that there are a possibility that the same effect can be obtained even if the magnetic field gradient is less than 2.5 mT/mm, and, further, a possibility that, if it exceeds 7 mT/mm, side effects such as excessive lowering of blood pressure and the maintaining the effect are exhibited, conversely.

Therefore, it is defined that the magnetic field gradient range in which the stellate ganglion magnetic stimulation device and method disclosed in this embodiment provide a safe effect in the stellate ganglion is about 1.5 mT/mm or more and less than 7 mT/mm (20 mT to 150 mT for magnetic flux density).

In the above, though the typical embodiments of the present invention have been described, the present invention is not limited to these, and various design changes are possible, and all of these design changes are included in the technical scope of the present invention.

For example, it is possible to use an electromagnet as the magnet 3 (that is, to form a dynamic magnetic field). By changing the magnetic field on the body surface by using a coil, a current is generated in the living body non-invasively to stimulate nerves. This may also include mechanisms aimed at magnetic amplification. That is, the low-frequency magnetic stimulation to the stellate ganglion G can suppress the nerve activity. It is also possible to use a magnet of embedded type.

The position of the stellate ganglion G may be determined from the position of the first thoracic vertebra instead of the seventh cervical vertebra or in addition to the seventh cervical vertebra.

### INDUSTRIAL APPLICABILITY

The stellate ganglion magnetic stimulation device according to the present invention is suitably used, for example, for suppressing or alleviating the hot flash symptoms seen in menopausal women, and, needless to say that it can be applied to symptoms other than the hot flashes. The main cause of the hot flash symptoms is abnormal excitation of the sympathetic nerves caused by abnormalities in the body temperature center associated with the decline of female hormones, and the stellate ganglion magnetic stimulation device according to the present invention suppresses the sympathetic nerves. In this regard, although there include hormone replacement therapy, SSRIs, Chinese herbal medicines and equol as the known treatments, none of them satisfy both high efficacy and safety.

Further, when the stellate ganglion magnetic stimulation device according to the present invention is applied to symptoms other than the hot flashes, for example, in addition to the stellate ganglion, it seems possible to be targets to be stimulated such as the maxillary ganglion located between C2 and C3, and the middle cervical ganglion located at C6 in FIG. 1, the vertebral artery ganglion located between the middle cervical ganglion and the stellate ganglion, and all of other systemic nervous systems in whole body.

### EXPLANATION OF SYMBOLS

1 Stellate ganglion magnetic stimulation device
2 Head unit
3 Magnet
4 Neck unit

## Claims

1. A stellate ganglion magnetic stimulation device, which comprises:
a head unit having a magnet for generating magnetism; and
a neck unit that is detachably worn around a neck portion, ear portion or head portion of a wearer and that presses the head unit against epidermis corresponding to the stellate ganglion so that stimulation by the magnetism acts on the stellate ganglion.

2. The stellate ganglion magnetic stimulation device according to claim 1, wherein
the magnet includes at least two magnet pieces, and
the at least two magnet pieces are so arranged as to satisfy the equation of 30°≤α≤180° where α is the angle between their axes.

3. The stellate ganglion magnetic stimulation device according to claim 1, wherein
the magnet includes at least three magnet pieces, and
the at least three magnet pieces are so arranged as to satisfy the equations of 30°≤β≤180° and 30°≤γ≤180° where 6 and y are the angles between the axes of adjacent magnet pieces.

4. The stellate ganglion magnetic stimulation device according to any one of claims 1 to 3, wherein
the magnet generates a magnetic field such that a magnetic flux density of 20 mT or more reaches the corresponding stellate ganglion.

5. The stellate ganglion magnetic stimulation device according to any one of claims 1 to 4, wherein
the neck unit has an expansion and contraction mechanism for expanding and contracting the neck unit along the circumference of the neck portion.

6. A method of wearing the stellate ganglion magnetic stimulation device according to any one of claims 1 to 5, which comprises the steps of
determining the position of the stellate ganglion based on the position of at least one of the seventh cervical vertebra and the first thoracic vertebra of the wearer, and
marking the epidermis corresponding to the determined position of the stellate ganglion,
wearing the neck unit around the neck portion, and
arranging the head unit according to the mark.

7. A method of using the stellate ganglion magnetic stimulation device according to any one of claims 1 to 5, which comprises the steps of:
determining the position of the stellate ganglion based on the position of at least one of the seventh cervical vertebra and the first thoracic vertebra of the wearer, and
marking the epidermis corresponding to the determined position of the stellate ganglion,
wearing the neck unit around the neck portion, and
arranging the head unit according to the mark.
